# EUROPEAN PATENT SPECIFICATION

(11) **EP 2 210 100 B1**
(45) Date of publication and mention of the grant of the patent: **01.06.2011**
(21) Application number: 08851189.4
(22) Date of filing: 21.11.2008
(51) Int. Cl.: G01N 33/542, G01N 33/574

(54) **IMPROVED FRET-PROBES AND USE THEREOF**
VERBESSERTE FRET-SONDEN UND IHRE VERWENDUNG
SONDES FRET (FLUORESCENCE PAR TRANSFERT D'ÉNERGIE DE RÉSONANCE) AMÉLIORÉES ET LEUR UTILISATION

(30) Priority: 21.11.2007 US 4112 P
(43) Date of publication of application: 28.07.2010
(73) Proprietor: Erasmus University Medical Center Rotterdam, 3015 GE Rotterdam (NL)
(72) Inventor: VAN DONGEN, Jacobus Johannes Maria, NL-3015 GE Rotterdam (NL); ORFAO DE MATOS CORREIA E VALE, José Alberto, NL-3015 GE Rotterdam (NL)
(74) Representative: Habets, Winand
(86) International application number: PCT/NL2008/050737
(87) International publication number: WO 2009/067009

(56) References cited:
- EP-A- 1 382 965
- WO-A-2004/042398
- WO-A-2004/042404

## Description

This invention relates to the detection of, among others, tumor-specific fusion proteins and protein interactions. More specifically, the invention relates to techniques that indicate the presence of a fusion protein and/or interacting proteins at the single cell level.

The diagnosis and classification of malignancies is frequently based on the detection of specific protein molecules or sets of protein molecules, as well as the detection of oncogenetic aberrations, mainly at the DNA level or RNA level [1]. The current genomics and proteomics studies in normal and malignant cells are drastically extending the information about gene expression and genetic aberrancies. This leads to the discovery of multiple new protein networks, which regulate cell-cell interactions, cell activation, signaling pathways, proliferation, differentiation, apoptosis and many other normal and abnormal cellular functions. Unravelling these protein networks requires the specific detection of true co-localization of the individual protein molecules.

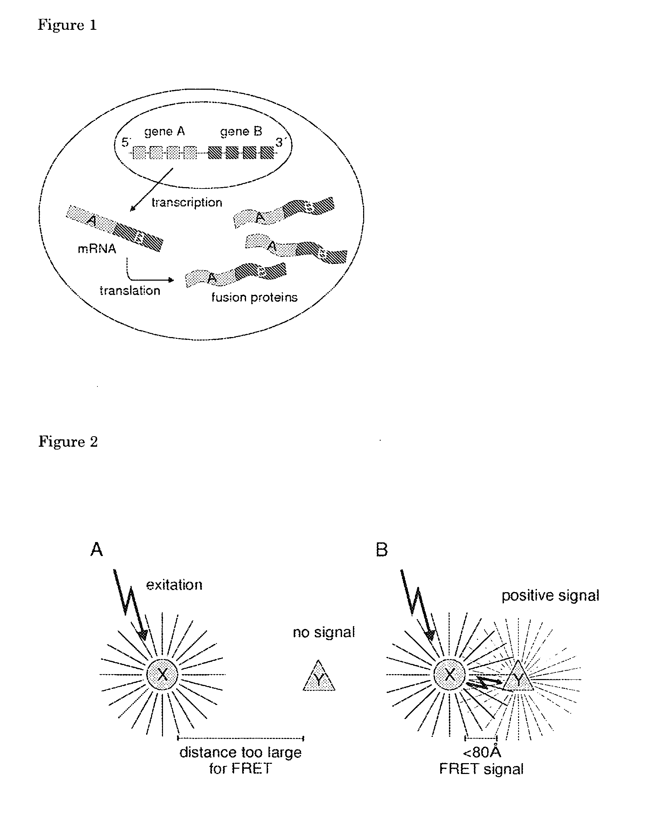

Classification of malignancies is particularly based on cell lineage and differentiation characteristics and the presence of specific chromosome aberrations. Many of these chromosome aberrations result in fusion genes, i.e. aberrantly coupled genes with the upstream part of one gene coupled to the downstream part of the other gene and vice versa [2-5]. These fusion genes are transcribed into fusion gene transcripts and translated into fusion proteins (Figure 1), which are assumed to play an important role in the oncogenic process. So far, more than hundred different types of fusion genes have been described in leukemias, lymphomas, and solid tumors. Exemplary fusion proteins are listed in Table 1.

Consequently, the reliable detection of these tumor-specific fusion proteins at the single cell level would be a major step forward in the diagnosis and classification of cancer patients as well as for monitoring of the disappearance of the malignant cells during treatment as measure of the effectiveness of the applied therapy protocol.

| **TABLE 1. Examples of fusion proteins in malignancies³, which may be detected via the FRET-technology using the nucleotide linker system for close and stable juxtapositioning of differentially labeled antibodies** | | |
|---|---|---|
| **Malignancy** | **Chromosome aberration** | **Fusion protein** |
| Chronic myeloid leukemia | t(9;22)(q34;q11) | BCR-ABL |
| Lymphoma | t(2;5)(p23;q35) | NPM-ALK |
| Prostate cancer | t(21;21)(q22.3;q22.2) | TMPRSS2-ERG |
| Ewing sarcoma | t(11;22)(q24;q12) | EWSR1-FLI1 |
| Papillary renal cell carcinoma | t(X;1)(p11;q23) | PRCC-TFE3 |
| Follicular thyroid carcinoma | t(2;3)(q13;p25) | PAX8-PPARG |
| Fibromyxoid soft tissue sarcoma | t(7;16)(q33;p11) | FUS-CREB3L2 |
| Endometrial stromal carcinoma | t(7;17)(p15;q11) | JAZF1-SUZ12 |
| Soft tissue chondrosarcoma | t(9;22)(q31;q12) | EWSR1-NR4A3 |
| Desmoplastic small round cell tumor | t(11;22)(p13;q12) | EWSR1-WT1 |
| Poorly differentiated carcinoma affecting midline structures | t(15;19)(q14;p13) | BRD4-NUT |

Initially, scientists tried to raise fusion-protein specific antibodies by making antibodies against the fusion epitopes of the fusion proteins. This approach has rarely been successful and if specific antibodies were obtained, they generally were not applicable in fluorescence microscopy or flow cytometry [6-8]. For example, the ER-FP1 antibody against the BCR-ABL p190 fusion protein works nicely in Western blotting, but was not successful in microscopic studies on human BCR-ABL positive leukemias [6,7].

Despite these initial problems, specific detection of fusion proteins has become possible via the application of a catching antibody against one part of the fusion protein and a labeled detection antibody against the other part of the protein. In such systems, the catching antibody is bound to a solid layer, such as a dipstick, an ELISA plate, or beads that can be analyzed by flow cytometry [9]. Although elegant and easy to perform, these systems use cell lysates and consequently do not allow detection of intracellular fusion proteins at the single cell level.

A close interaction between two different membrane-bound and/or intracellular proteins, or the presence of fusion proteins can be investigated by use of antibodies that are conjugated with or linked to fluorochromes that are suited for fluorescence resonance energy transfer (FRET). FRET technology is based on the juxtapositioning of two different fluorochromes (with different excitation wavelengths) of which one fluorochrome produces emission light that excites the other fluorochrome (Figure 2). If the emission light of the second fluorochrome can be detected by flow cytometry, this allows easy, highspeed analysis of protein networks and detection of fusion proteins at the single cell level. Using e.g. confocal laser scanning microscopy, it becomes possible to evaluate the precise subcellular position of the interacting protein and fusion proteins.

Approximate colocalization of two FRET fluorochrome-conjugated antibodies is not sufficient for the required light-energy transfer. True colocalization of the detected proteins is needed so that close juxtapositioning of the fluorochromes linked to two different antibodies occurs (generally < 80 Å, but preferably < 50 Å, most preferably < 10 Å), which is essential for efficient light-energy transfer.

We previously described that FRET technology can be used to detect the presence of a fusion protein (WO2004/042398) or to detect protein interactions (WO2004/042404) in a cell using a set of specifically designed probes. According to WO2004/042398 and WO2004/042404, each probe is provided with a dye wherein said dyes together allow FRET, and at least one probe is provided with a reactive group. The addition of a "bridging" reagent capable of binding to the reactive groups allows juxtaposing the first and second probe such that there is an increased likelihood of energy transfer between the FRET dyes. W02004/042398 for example discloses a set of antibody probes A and B directed against fragments A and B of an A-B fusion protein, wherein A and B are labelled with different FRET dyes and wherein A and B were provided with biotin reactive groups capable of binding a (strept)avidin bridging substance. Upon addition of the bridging substance to the reactive groups, the spatial organization of the antibody probes is modulated via the reactive groups. This allows the individual FRET dyes that are attached to the probes to come within a distance of each other that allows FRET to occur, i.e. within about 80-100 Angstrom of each other. Whereas the probes and methods of WO2004/042398 and WO2004/042404 can generally yield satisfactory results, the present inventors sought to further improve the concept. In particular, it is a goal of the present invention to increase the sensitivity of FRET-based methods for detecting fusion proteins and interacting proteins.

This goal was met by the realization that oligonucleotide moieties are highly suitable to bring the FRET fluorochromes in close and stable juxtaposition, such that FRET can occur with great efficiency. The small size of oligonucleotides allows for only a minimal spacing between the juxtaposed dyes. Furthermore, complementary oligonucleotide sequences can be designed to achieve highly specific and strong intermolecular interactions. Thus, the inventors identified oligonucleotides (e.g. DNA or PNA or LNA molecules) as excellent reactive groups and bridging substance to mediate and/or enhance close juxtapositioning of dye-labeled probes.

The invention in its broadest scope is defined in independent claim 1:

A set of at least a first and a second molecular probe, each probe capable of specifically binding to a molecule of interest via its binding domain, each probe provided with a dye wherein said dyes together allow energy transfer, each probe additionally provided with a reactive group allowing juxtaposing said at least first and second probe wherein said reactive group remains available for modulating the spatial organization of juxtaposed probes after the probe is bound to a molecule of interest, wherein said reactive group comprises an oligonucleotide and wherein the reactive group of said first probe is not directly reactive with the reactive group of said second probe and requires a bridging substance capable of bridging at least two reactive groups to mediate and/or enhance close juxtaposing of said probes.

The reactive group of said first probe is not directly reactive with the reactive group of said second probe. This is required to avoid false-positive signals generated by unwanted self-association between the probes.

Independent claims 8, 10, 12, 17 and 19 all refer back to claim 1. Preferred embodiments are defined in dependent claims 2-7, 9, 11, 13-16 and 18.

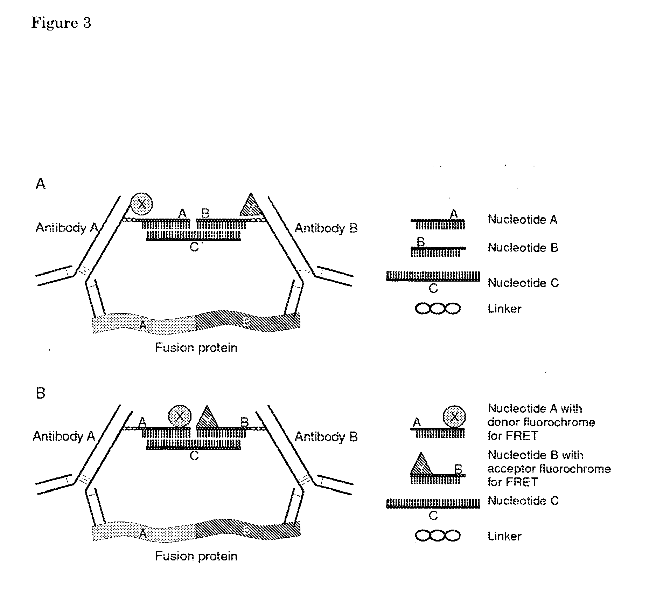

The principle underlying the present invention is schematically illustrated in Figure 3. A first molecular probe (antibody A) is provided with FRET dye X and with at least one reactive group, said reactive group comprising or consisting of an oligonucleotide (Nucleotide A). The reactive group is capable of binding specifically to a bridging substance (Nucleotide C) comprising or consisting of a nucleic acid sequence of which a part is complementary to the sequence of at least a fragment of Nucleotide A.. Nucleotide C is also complementary to at least part of the sequence of the reactive group (Nucleotide B) of a second molecular probe (Antibody B) provided with FRET dye Y. The dyes X and Y together form a FRET pair. Only if the first and second probe come into close proximity of each other (e.g. because they are bound to adjacent epitopes on a fusion protein A-B as shown in the figure, or to epitopes on interacting molecules), the oligonucleotide reactive groups (Nucleotides A and B) are sufficiently close together for the bridging substance (Nucleotide C) to bind to the reactive groups of both the first and second probe. This interaction will reduce and stabilize the distance between the two probe-bound dyes such that a FRET signal can be detected.

FRET energy transfer efficiency is inversely proportional to the sixth power of the distance between the donor dye and the acceptor dye. The very small size of the oligonucleotide reactive groups and bridging substance of the oligonucleotide linker system as disclosed herein allows a very close proximity of the dyes (e.g. within 10 Ångstrom), resulting in a much stronger fluorescence signal as compared to using the proteinaceous reactive groups and bridging substance as disclosed in WO2004/042398 or WO2004/042404. Furthermore, the base-pair recognition between the complementary sequences of the reactive group and the bridging substance, yet not between the reactive groups themselves, provides a high degree of specificity.

In this FRET approach with three oligonucleotide molecules as stabilizing linker system, cells are typically first subjected to the intracellular labeling with the at least two probes each carrying an oligonucleotide reactive group, followed by (stringent) washing and subsequent incubation with the specifically designed bridging oligonucleotide to obtain close and stable linkage of the two reactive antibodies. In a preferred embodiment, each of the probes is provided with a multiplicity of reactive groups, like 2-6 oligonucleotides each being reactive with a bridging oligonucleotide. Said reactive groups present on a single probe may be the same or different to each other.

As used herein, the expressions "reactive group oligonucleotide" and "oligonucleotide reactive group" are used interchangeably, unless indicated otherwise. Also, "bridging oligonucleotide" and "oligonucleotide bridging substance" refer to the same entity.

The term "oligonucleotide" as used herein refers to a stretch of nucleic acids or nucleic acid analogs joined in a long chain. The total length of the oligonucleotide can vary, depending among others on the nature of the nucleic acid or nucleic acid analog. In one embodiment, an oligonucleotide consists of a stretch of 5-50, preferably 10-30 nucleic acids or nucleic acid analogs joined in a long chain. A nucleic acid is for instance a nucleotide comprising a nitrogenous base (A, G, T, or C in DNA; A, G, U, or C in RNA), a charged phosphate moiety, and a sugar moiety (deoxyribose in DNA and ribose in RNA).

Suitable lengths for the probe-bound oligonucleotides (i.e. the reactive groups) include those consisting of at least 8 nucleic acid residues, preferably 10-18 nucleic acids or analogs. The lengths of the oligonucleotide reactive groups on the respective probes may be different or the same. In one embodiment, they are of the same length, such as 10-15, preferably 10-12 nucleic acids or analogs. The bridging oligonucleotide will generally be longer than the reactive group oligonucleotides. In one embodiment, the bridging or linker oligonucleotide consists of 15-40 nucleic acids or analogs thereof, for example 18-30, like from about 20 to about 25.

In a preferred embodiment, the oligonucleotide is a peptide nucleic acid (PNA) oligomer. PNA is similar to DNA or RNA but differs in the composition of its "backbone." DNA and RNA have a deoxyribose and ribose sugar backbone, respectively, whereas PNA's backbone is composed of repeating N-(2-aminoethyl)-glycine units linked by peptide bonds. The various purine and pyrimidine bases are linked to the backbone by methylene carbonyl bonds. PNAs are typically depicted like peptides, with the N-terminus at the first (left) position and the C-terminus at the right.

The nucleic acid analog PNA is not known to occur naturally in existing life on earth, but it can be artificially synthesized. It has been used in certain areas of biological research and medical treatments. Synthetic peptide nucleic acid oligomers have been used in recent years in molecular biology procedures, diagnostic assays and antisense therapies. Since the backbone of PNA contains no charged phosphate groups, the binding between PNA/DNA strands is stronger than between DNA/DNA strands due to the lack of electrostatic repulsion. Early experiments with homopyrimidine strands (strands consisting of only one repeated pyrimidine base) have shown that the Tm ("melting" temperature) of a 6-base thymine PNA/adenine DNA double helix was 31°C in comparison to an equivalent 6-base DNA/DNA duplex that denatures at a temperature less than 10°C. Mixed base PNA molecules are true mimics of DNA molecules in terms of base-pair recognition.

PNA oligomers also show greater specificity in binding to complementary DNAs, with a PNA/DNA base mismatch being more destabilizing than a similar mismatch in a DNA/DNA duplex. This binding strength and specificity also applies to PNA/RNA duplexes. PNAs are not easily recognized by either nucleases or proteases, making them resistant to enzyme degradation. PNAs are also stable over a wide pH range. Finally, their uncharged nature makes crossing through cell membranes easier, which may further improve their value for the present invention which involves detection of a fusion protein in intact cells. In one aspect, a PNA oligonucleotide consisting of about 10 to 16, like 12-15 PNA units is used as reactive group oligonucleotide, optionally in combination with a bridging oligonucleotide consisting of 20-30 PNA units. In a specific aspect, the probe-bound PNA sequences each consist of 10-12 PNA units complementary to a bridging oligonucleotide consisting of 20-25, like 21, PNA units.

In yet another embodiment, the oligonucleotide comprises Locked Nucleic Acids (LNA™). LNA is a novel type of nucleic acid analog that contains a 2'-O, 4'-C methylene bridge. This bridge-locked in 3'-endo conformation-restricts the flexibility of the ribofuranose ring and locks the structure into a rigid bicyclic formation, conferring enhanced hybridization performance and exceptional biological stability.

As will be understood by a person skilled in the art, for the reactive groups and bridging substance various different combinations of types of nucleic acid oligomers (e.g. DNA, RNA, LNA, PNA) can be used. The specific, high affinity interaction between reactive group and bridging substance can be effected through either homoduplex (e.g. DNA/DNA, PNA/PNA) or heteroduplex (e.g. DNA/PNA) formation. In one embodiment, a probe set of the invention comprises at least a first and a second probe, each probe provided with a distinct oligonucleotide as reactive group, wherein said nucleic acid oligomer is a deoxyribonucleotide oligomer (DNA). These DNA reactive groups can be clustered by different types of bridging substances, for instance a DNA (homoduplex) or a PNA (heteroduplex) bridging substance. Alternatively, the reactive groups comprise an oxyribonucleotide sequence (RNA) which can be recognized and bound by an RNA (homoduplex) or PNA (heteroduplex) bridging substance. It is also possible to use a homoduplex between a bridging substance and the reactive group of an at least first probe and a heteroduplex between the bridging substance and the at least second probe. For example, probe A is provided with a PNA reactive group and probe B with a DNA or RNA reactive group, both groups capable of being clustered by a PNA bridging substance.

The extent or degree of complementarity between the bridging oligonucleotide and either one of the oligonucleotide reactive groups can vary, as long as it allows for a specific and stable binding. In one embodiment, there is complementarity (i.e. base-pairing) between a reactive group and a bridging substance over a stretch of at least 5, preferably at least 7 consecutive nucleic acids or analogs. As will be understood, the oligonucleotide reactive group of the first probe is not directly reactive with the oligonucleotide reactive group of the second probe in order to avoid self association of the probes and premature energy transfer to occur between the attached dyes. This is important to ensure that a FRET signal truly reflects juxtaposed probes.

In one embodiment, the bridging substance comprises a first sequence that is complementary to at least part of a first oligonucleotide reactive group and a second sequence that is complementary to at least part of a second oligonucleotide reactive group, wherein the first and second sequence are separated by at least one nucleic acid or analog. For example, they are spaced by a few e.g. 1-10 such as 2, 3, 4, or 5 nucleic acids. A spacing of 1-3 is preferred. In another embodiment, the complementary sequences are spaced by one or more amino acids residues, preferably 1-2 small amino residues like glycine residues.

However, it is also possible that the sequence complementary to at least part of an oligonucleotide reactive group of a first probe is flanked directly, without spacing, by the sequence complementary to at least part of an oligonucleotide reactive group of a second probe. It is preferred that both termini of the bridging oligonucleotide are designed to participate in the binding to the oligonucleotide reactive groups, such that there are no single stranded "free ends".

In addition, the oligonucleotide sequences should be selected such that they are not cross-reactive with endogenous nucleotide sequences of the cell in which the fusion protein is to be detected. Thus, when designing any of the sequences used for practising the invention, complementarity with endogenous (e.g. human) DNA and/or RNA sequences should be minimized or even completely avoided in order to prevent unwanted blocking or scavenging of the oligonucleotides.

In one embodiment, a first probe is provided, e.g. via a linker, with a reactive group oligonucleotide consisting of the sequence 5'-CGA TTC TAT G-3' and a second probe being provided, e.g. also via a linker, with a reactive group oligonucleotide comprising the sequence 5'-TGT ACC TTG A-3'. This set of probes is advantageously used in combination with a bridging oligonucleotide comprising or consisting of the sequence 5'-TCA DGG TAC A Gly Gly CAT AGA ATC G-3'. The skilled person will however understand that the present invention can be practiced using any set of sequences, be it DNA, RNA, PNA or any combination thereof, that allows for sufficient binding strength and binding specificity between the bridging substance and the respective probes.

A molecular probe is capable of specifically binding to a biological molecule of interest via its so-called binding domain. Following binding of at least a first and a second probe to a molecule of interest via the binding domain, a reactive group is used to modulate juxtapositioning. An oligonucleotide reactive group remains available for modulating the spatial organization of juxtaposed probes after the probe is bound to a molecule of interest. In one embodiment, said molecule of interest is a protein, preferably a fusion protein, more preferably an oncogenic fusion protein. Particularly preferred is a set of a first and a second molecular probe wherein each probe is capable of recognizing and binding to a binding site (epitope) positioned at opposite sides of the fusion region of said fusion protein. Of course, when using a set of probes wherein each probe binds to a different epitope of a molecule of interest (e.g. epitopes at the C- and N-terminal side of the fusion region of a fusion protein), said different epitopes should not interact with each other in either an inter- or intramolecular fashion because this would obviously interfere with probe binding. Different probes within a set of probes are therefore capable of binding to different, essentially non-interacting epitopes. Provided that the probes recognize binding sites (epitopes) within a small distance of each other, the mere binding of the probes to a fusion protein or to interacting molecules could, in theory, give rise to energy transfer between the dyes. However, by the "clustering" of juxtaposed reactive groups by a bridging substance the spatial organization of the dyes can be modulated such that the likelihood of energy transfer is dramatically enhanced.

The present invention also provides a diagnostic kit comprising a set of probes according to the invention. In a preferred embodiment, the kit additionally comprises an oligonucleotide bridging substance which has a sequence that is complementary to at least part of the oligonucleotide reactive group of the first probe, and which is complementary to at least part of the oligonucleotide of the second probe. For example, such a kit may be used for monitoring and quantification of malignant cells, e.g. leukemic cells, via the detection of tumor-specific fusion protein-positive cells. The diagnostic test kit provided herein is useful at the time of diagnosis as well as during and after treatment to evaluate the effectiveness of the applied cancer treatment protocol.

A further aspect relates to a method using a set of probes for detecting the presence of a fusion protein or interacting (proteinaceous) molecules in the diagnosis and / or classification of a disease as well as before, during and after treatment of a disease to evaluate the effectiveness of said treatment

Also provided is a method for producing a probe set according to the invention comprising contacting each probe with an oligonucleotide reactive group to form a conjugate between said probe and said reactive group and purifying said conjugate. The reactive group oligonucleotide may be attached to the probe directly or indirectly, for instance via spacer or linker moiety. Also, the FRET dye can be attached to the probe directly or indirectly, e.g. via the reactive group. In a preferred embodiment, a probe comprises at least one oligonucleotide reactive group, which reactive group is provided with a FRET dye (see Fig. 3B). The oligonucleotide reactive group may be coupled directly or indirectly to the probe. The reactive group may be provided with a FRET dye prior to or after its conjugation to a probe.

In a preferred embodiment of the invention, a probe set comprises a set of at least two dye-oligonucleotide-conjugated antibodies, each antibody capable of recognizing a binding site positioned at opposite sides of the fusion region of a fusion protein or at distinct interacting molecules, e.g. proteins in a protein complex. A suitable antibody comprises a conventional (poly- or monoclonal) or a synthetic antibody or a binding fragment functionally equivalent thereto, such as a Fab', Fab, a single chain Fv fragment, a diabody (a single chain Fv dimer) and the like. For example, a chimeric fusion protein A-B can be detected via FRET using a set of dye-conjugated probes, e.g. an anti-A antibody and an anti-B antibody. In a preferred embodiment, a sample is contacted with two antibodies, one against domain A and the other against domain B of a fusion protein to detect the presence of an A-B fusion protein in a cell sample. One antibody is labelled (preferably via its reactive group) with a FRET donor dye and an other with a FRET acceptor dye. Only when domain A is in close proximity to domain B, e.g. when both are part of the same protein molecule, the two antibodies become sufficiently close together ('juxtaposed') which allows the donor/acceptor pair to induce a detectable FRET fluorescence signal.

In the present context, the term "reactive group" refers to a moiety which allows modulating the spatial organization of FRET dyes such that there is an increase in the probability of energy transfer to occur and / or an increase in energy transfer efficiency. The spatial organization refers to both the distance between the dyes as well as to their relative orientation. Modulating the spatial organization includes adjusting and stabilizing the spatial organization of dyes. One of the primary conditions for energy transfer to occur is that donor and acceptor molecules must be in close proximity, typically 10-100 Å. In a preferred embodiment, a reactive group allows juxtaposing said dyes within a distance of 50 Å of each other, more preferably within 20 Å of each other but most preferably within a distance of 10 Å of each other.

In the present context, the term "dye" refers to a substituent which, in concert with another dye, can be used for energy transfer analysis, such as FRET analysis. As mentioned above, FRET is usually based on the interaction between donor and acceptor dyes that are both fluorescent. In one embodiment, the invention uses a set of probes wherein at least one of said dyes is a fluorochrome. However, a nonfluorescent acceptor may also be used and FRET is detected by quenching of donor fluorescence. As said, detecting FRET by monitoring a decrease in donor fluorescence as a consequence of juxtapositioned probes is often not as sensitive as detecting in increase in acceptor fluorescence. Thus, in a preferred embodiment, at least two fluorescently labeled probes are used to detect a fusion protein, as is exemplified in the detailed description. Examples of preferred fluorochromes are those suitable for analysis by conventional flow cytometry and include fluorescein labels, e.g. 5-(and 6)-carboxyfluorescein, 5- or 6- carboxyfluorescein, 6-(fluorescein)-5-(and 6)-carboxamide hexanoic acid and fluorescein isothiocyanate, AlexaFluor™ dyes such as AlexaFluor 488™ or AlexaFluor 594™, cyanine dyes such as Cy2, Cy3, Cy5, Cy7, optionally substituted coumarin, R-phycoerythrin, allophycoerythrin, Texas Red and Princeston Red as well as conjugates of R-phycoerythrin and, e.g. Cy5 or Texas Red and members of the phycobiliproteins. Other dyes of interest are quantum dot dyes, which come in a nearly unlimited palette of colours. Extensive information on donor/acceptor pairs suitable for energy transfer detection by flow cytometry can be found in Szollosi et al.¹⁸ Referred combinations of fluorochromes comprise those dyes used in the classical tandem conjugates, also referred to as duochromes ¹⁹. In a preferred embodiment, probes are provided with a set of dyes that are used in LightCycler technology, such as fluorescein in combination with LCRed640™ or LCRed705™.

Also provided herein is a method for detecting the presence of a fusion protein in a cell using a set of at least a first and a second molecular probe, each probe capable of recognizing a binding site (via its binding domain) positioned at opposite sides of the fusion region of said fusion protein, each probe further provided with a dye wherein said dyes together allow energy transfer, at least one probe provided with an oligonucleotide reactive group allowing to modulate juxtaposing said at least first and said second probe such that there is an increased likelihood of energy transfer between said dyes, comprising providing a set of probes, providing a sample comprising a cell, contacting said sample with said probes under conditions that allow juxtaposing said probes on said fusion protein, removing any unbound and any non-specifically bound probe and detecting juxtaposition of said probes via FRET to determine the presence of said fusion protein.

In one embodiment, a probe is provided with more than one oligonucleotide reactive group, enabling said probe to interact with more than one bridging substance. Providing a probe with more than one reactive group will theoretically increase the likelihood of an interaction between said probe and a bridging substance.

Next, the invention provides a method for detecting a fusion protein at the single cell level using of a set of probes according to the invention, each probe capable of binding to a binding site positioned at opposite sides of a fusion region of said fusion protein via the binding domain of the probe i.e. one probe is directed against a protein fragment comprising the N-terminal fragment of a fusion protein, and an other probe is directed against a protein fragment comprising the C-terminal fragment of the same fusion protein. A fusion protein comprises any kind of proteinaceous substance which is formed after transcription and translation of a fusion gene. A fusion gene comprises one part of one or more genes combined with another gene or a part derived thereof. A fusion protein may be the result of a chromosomal translocation, inversion or deletion. In a preferred embodiment, a method provided is used to detect a tumor-specific fusion protein. A fusion protein may be an endogenously expressed protein or it may be the result of genetic engineering. Fusion proteins in malignancies which can readily be detected using a method according to the invention include but are not limited to those listed in Table 1.

Many different applications could be envisaged using the proposed oligonucleotide-based FRET method disclosed herein, for example:
- Detection of natural and oncogenic fusion proteins :
   - oncogenic fusion proteins occurring in several leukemias and solid tumors
   - T-cell receptor or B-cell receptor proteins formed by fusions of gene segments
- Detection of association of specific T-cell receptor chains (e.g. a Vδ2⁺ with a Vγ9⁺ chain);
- Investigation of protein complexes: Are all components of a protein complex present? How close are the components linked?
- Investigation of gene regulation by transcription complexes (e.g. the AML1/CBFβ core binding factor transcription complex, that is a critical regulator of normal hematopoiesis);
- Investigation of activation of transcription by protein complexes (e.g. binding of β-catenin to Tcf-1 induces transcription of Wnt-target genes);
- Detection of protein-DNA or protein-RNA interactions, for investigation of proteins involved in transcription or translation;
- Evaluation of cell-cell interactions via antibodies directed against different cell types involved in the same interaction process.

The method disclosed herein has several advantages for application in tissue sections and smears:
- application in parallel to other immunohistochemical stainings
- combination with split-signal FISH: detection of oncogenic events at DNA level (fusion gene) and at the protein level (fusion protein).

It is of great relevance to note that the present method does not require disruption of the cell integrity, e.g. the preparation of a cell lysate, to detect the presence of an intracellular fusion protein or molecular complex. Preservation of the morphology integrity of a cell permits analysis at the single cell level, for example by flow cytometry or fluorescence microscopy. Detection of a FRET signal by flow cytometry offers the ability to perform rapid, multiparametric analysis of specific individual cells in a heterogeneous population. The main advantage of flow cytometry is that it directly gives quantitative data and that it is very rapid (results can be obtained in a few hours).

The method provided in the present invention allows detection of a fusion protein or interacting molecules at the single cell level. A sample comprising a cell can be treated so as to obtain a permeabilisation of the material and a preservation of the morphology. The preferred treatment is one which fixes and preserves the morphological integrity of the cellular matrix and of the proteins within the cell as well as enables the most efficient degree of probe, e.g. antibody, penetration.

Unlike for example a 'catching/detection' antibody method, which can essentially only be applied to detect the presence of a fusion protein or interacting proteins at the cell surface or in a cell lysate, the present method allows gating of subset of cells that are present in a mixture of cells via immunophenotypic characteristics. Consequently, the method provided herein permits detection in a rare population of malignant cells in a large background of normal cells. This is especially advantageous for detecting low frequencies of fusion-positive cells like in the case of detection of minimal residual disease (MRD) during or after treatment for evaluation of treatment effectiveness. In preferred embodiment, the method provided includes multiparameter flow cytometry to identify and / or isolate single cells to detect the presence of a fusion protein at the single cell level. All that is required for practicing the method provided is a flow cytometry facility. Importantly, the procedure can be performed in routine laboratories by personnel with ordinary skills.

More than a hundred different fusion genes and fusion proteins have been described in various types of cancer. As said, the method provided allows to discriminate between the presence of normal proteins and an aberrant fusion protein at the single cell level. Theoretically, two antibodies recognizing two different domains of a fusion protein can cause a background staining by binding to the domains on the normal proteins that are derived from the normal genes instead of the fusion gene. However, in certain cases only one of the two normal proteins reaches a detectable expression level in a target cell population, as defined by cell surface and / or intracellular markers. Furthermore, the normal proteins and the fusion protein often differ in their intracellular expression pattern, frequently resulting in a different subcellular localization. This implies that coincidental colocalisation of the two different normal proteins is unlikely to occur at a significant level. In particular, coincidental juxtaposing probes sufficient for a FRET signal will be rare in normal cells, if this occurs at all.

The method provided comprises providing a sample comprising a cell, whereby said sample is optionally subject to fixation and permeabilization. A sample may comprise a primary cell that is obtained from a biological sample. A biological sample can be a body fluid sample including blood, serum, urine, bone marrow, cerebrospinal fluid (CSF), saliva. It may also be a tissue sample, tissue homogenate. A sample comprises a cultured cell which may be a cultured primary cell, for example tumor cells obtained from a lymph node biopsy. Furthermore, a sample may comprise a cultured cell from an established laboratory cell line, like a K562, KASUMI-1, REH or CEM cell line, which can be obtained from a number of sources such as the American Type Culture Collection (ATCC; www.atcc.org for an online catalog) or the German Collection of Microorganisms and Cell Cultures (DSMZ; www.dsmz.de for an online catalog). The method provided is suitable to detect the presence of an endogenous fusion protein as well as a recombinant fusion protein in a cell. The method provided is also suitable to detect interactions between recombinant proteins and/or endogenous molecules in a cell.

For analysing a sample comprising a suspension of cells, it is preferred that the sample is treated so as to obtain a preservation of the morphology of the material and permeabilisation in order to ensure sufficient accessibility of a molecule of interest to a probe. The type of treatment will depend on several factors, for instance on the fixative used, the extent of fixation and the type and properties of the molecule of interest. Fixation may be carried out with a fixative such as formaldehyde.

For the detection of in primary cells, it is especially advantageous to use an additional marker to define a target cell population of interest. A number of important biological applications in infectious diseases, MRD detection and monitoring, and gene therapy typically require the analysis and isolation of rare cells (e.g. haemopoietic stem/progenitor cells) from a large "background" of other cells. In one embodiment of the invention, the method includes staining a sample for at least one cellular marker, like a cell surface marker or an intracellular marker, to define a target cell population within a mixture of cells comprising contacting said sample with a compound capable of selectively binding to said marker. In a preferred embodiment, such a compound is directly tagged with a fluorescent dye. A suitable compound comprises a fluorescently labelled antibody or a binding fragment functionally equivalent thereto. Also, a compound capable of selectively binding to a cellular marker can be used which can be detected using a dye-conjugated secondary reagent (e.g. a fluorescently labelled secondary antibody). A cellular marker comprises any kind of intracellular or membrane-bound marker which can be used to distinguish a subpopulation of cells in a mixture of cells. A mixture of cells comprises living cells. It also comprises permeabilized and / or fixed cells. A cellular marker can be a cluster of differentiation (CD) antigen. CD markers are cell surface molecules of among others haemopoietic cells that are distinguishable with monoclonal antibodies. Haemopoietic cells comprise thymocytes, dendritic cells, Langerhans' cells, neutrophils, eosinophils, germinal centre B cells, follicular dendritic cells, plasma cells and bone-marrow cells. For example, suitable cellular markers comprise CD1, CD3, CD4, CD8, CD10, CD19, CD20, CD33, CD34 and CD117. Monoclonal antibodies directed against a large number of human CD markers can be obtained from various suppliers, such as BD Biosciences or Ancell Immunology Research Products, Bayport, USA. Often, antibodies are available that are directly conjugated with a fluorochrome of choice e.g. CD10-PE or CD19-FITC, which is obviously a preferred choice to practice a method according to the invention.

In a preferred embodiment, a method is provided to identify and/ or isolate rare single cells using multiparameter flow cytometry/cell sorting techniques and to further characterize these cells by the presence or absence of a fusion protein of interest or by the identification of interacting molecules. Such a method is particularly suited for application to a number of important problems in immune system development, infectious diseases, cancer and gene therapy. Typically, prior to staining a cell sample with a probe set, cells are labeled with at least one relevant dye-conjugated antibody according to standard procedures in order to define a target cell population. The choice of dye should preferably, but not exclusively, aim at the usage of two or three dyes for immunophenotyping in addition to the FRET dyes. For example, a FRET probe set according to the invention can be combined with another dye to mediate leukocyte subset gating via immunophenotypic characteristics, e.g. CD10, CD19 and CD20 to accurately define subsets of precursor-B-cells in bone marrow, or CD1, CD4 and CD8 to define subsets of thymocytes, or CD34 and / or CD117 to identify stem/precursor cell populations. As shown herein in the detailed description, the invention provides a method which allows the detection of an intracellular fusion protein in a very small subset of cells, i.e. detection of MRD, which is essential for evaluating effectiveness of cancer treatment.

### Figure legends

**Figure 1****.** Schematic diagram of a fusion gene consisting of the upstream (5') part of gene A and the downstream (3') part of gene B. This A-B fusion gene is transcribed into A-B mRNA and translated into an A-B fusion protein.
**Figure 2****.** Schematic diagram of the principle of fluorescence resonance energy transfer (FRET) with fluorochrome X as donor dye and Y as acceptor dye. A. The acceptor dye Y will not be excitated by the emission light of the donor dye X, if the distance between X and Y is too large. B. If the distance between the donor and acceptor dye is sufficiently small (< 80 Ångstrom but preferably < 50 Ångstrom), the emission light of the donor dye X will excitate the acceptor dye Y.
**Figure 3****.** Schematic diagram depicting the use of oligonucleotides (e.g. DNA/PNA) molecules to closely and stably link two antibodies. When both antibodies come into close proximity of each other because they are bound to both partners of a fusion protein A-B, bridging substance oligonucleotide C, which is partly complementary to both oligonucleotide A and B, will reduce and stabilize the distance between the two fluorochromes X and Y, and a FRET signal can be detected. A. The donor and acceptor fluorochromes are conjugated directly to the antibody probes. B. The donor and acceptor fluorochromes are conjugated to the oligonucleotide reactive groups. The oligonucleotide reactive groups are attached to the antibody probes via a linker moiety.

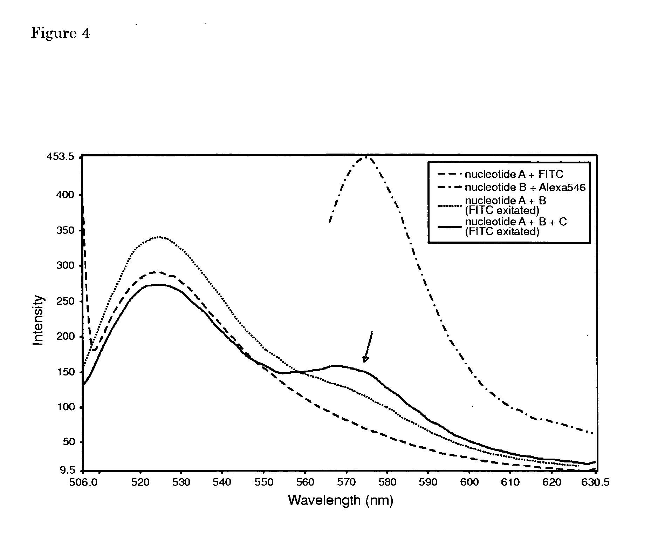

**Figure 4****.** shows the results of the fluorescence detected in the case of either reactive group oligonucleotide A, reactive group oligonucleotide B, combination of reactive group oligonucleotides A and B, or combination of A and B and bridging oligonucleotide C. The arrow indicates the FRET signal induced by the addition of complementary bridging oligonucleotide C. For details see the Example below.

### EXAMPLE

The following oligonucleotides were synthesized according to standard procedures:
Reactive group Oligonucleotide A: Linker CGA TTC TAT G Fluorescein
Reactive group Oligonucleotide B: Alexa-TGT ACC TTG A-linker
Bridging Oligonucleotide C: TCA DGG TAC A Gly Gly CAT AGA ATC G
10 pmol PNA of the different oligonucleotides were mixed in 200 µL phosphate buffer pH 7,2, and their fluorescence was measured. The hybridisation was complete within 5 minutes when PNA oligonucleotide C was added. For results see Figure 4.
Instrument: Perkin Elmer LS 55
   Excitation wavelength:fluorescein: 485 nm, Alexa 546: 546 nm.
   excitation slit 5 nm emmision slit 10 nm.

### References

1. Jaffe ES, Harris NL, Stein H, Vardiman JW (eds), World Health Organization classification of tumours. Pathology and genetics of tumours of haematopoietic and lymphoid tissues. Lyon: IARC Press, 2001.
2. Van Dongen JJM, Macintyre EA, Gabert JA, et al, Standardized RT-PCR analysis of fusion gene transcripts from chromosome aberrations in acute leukemia for detection of minimal residual disease. Report of the BIOMED-1 Concerted Action: investigation of minimal residual disease in acute leukemia. Leukemia 1999; 13: 1901-28.
3. Mitelman F, Johansson B, Mertens F, The impact of translocations and gene fusions on cancer causation. Nat Rev Cancer 2007; 7: 233-45
4. Look AT, Oncogenic transcription factors in the human acute leukemias. Science 1997; 278: 1059-64.
5. Crans HN, Sakamoto KM, Transcription factors and translocations in lymphoid and myeloid leukemia. Leukemia 2001; 15: 313-31.
6. Van Denderen J, Hermans A, Meeuwsen T, et al, Antibody recognition of the tumor-specific bcr-abl joining region in chronic myeloid leukemia. J Exp Med 1989; 169: 87-98.
7. Van Denderen J, ten Hacken P, Berendes P, et al, Antibody recognition of the tumor-specific b3-a2 junction of bcr-abl chimeric proteins in Philadelphia-chromosome-positive leukemias. Leukemia 1992; 6: 1107-12.
8. Sang BC, Shi L, Dias P, et al, Monoclonal antibodies specific to the acute lymphoblastic leukemia t(1;19)-associated E2A/PBX1 chimeric protein: characterization and diagnostic utility. Blood 1997; 89: 2909-14.
9. Berendes P, Recognition of tumor-specific proteins in human cancer, Ph.D. Thesis, Chapter 8. Rotterdam: Erasmus University Rotterdam, 1997: 111-27.

## Claims

1. A set of at least a first and a second molecular probe, each probe capable of specifically binding to a molecule of interest via its binding domain, each probe provided with a dye wherein said dyes together allow energy transfer, each probe additionally provided with a reactive group allowing juxtaposing said at least first and second probe wherein said reactive group remains available for modulating the spatial organization of juxtaposed probes after the probe is bound to a molecule of interest, wherein said reactive group comprises an oligonucleotide and wherein the reactive group of said first probe is not directly reactive with the reactive group of said second probe and requires a bridging substance capable of bridging at least two reactive groups to mediate and/or enhance close juxtaposing of said probes.

2. A set according to claim 1, wherein the oligonucleotide reactive group of said first and second probe are independently selected from the group consisting of deoxyribonucleotide (DNA) oligomer, oxyribonucleotide (RNA) oligomer, locked nucleic acid (LNA®) and peptide nucleic acid (PNA) oligomer.

3. A set according to claim 1 or 2 wherein the dye is conjugated to the reactive group.

4. A set according to anyone of claims 1 to 3 wherein each of said probes is provided with a multiplicity of reactive groups.

5. A set according to anyone of claims 1 to 4 wherein said probe is an antibody or a binding fragment functionally equivalent thereto.

6. A set according to anyone of claims 1 to 5 wherein at least one of said dyes is a fluorochrome.

7. A set according to claim 6 wherein said fluorochrome is selected from the group consisting of fluorescein isothiocyanate (FITC), tetramethylrhodamine isothiocyanate (TRITC), Texas Red (TR), R-phycoerythrin (R-PE), allophycocyanin (APC), members of the phycobiliproteins, cyanine dyes such as Cy3, Cy5, Cy 5.5 or Cy7, , Alexa Fluor dyes, fluorescein, LightCycler dyes, such as LCRed640 or LCRed705, tandem conjugates of these fluorochromes, and quantum dot dyes.

8. A method for providing a set of probes according to any one of claims 1-7 comprising, contacting each probe with a suitable dye and with a reactive group comprising an oligonucleotide to form a conjugate between said probe, said dye and said reactive group.

9. Method according to claim 8, comprising conjugating the dye to the reactive group.

10. A method for detecting the presence of a fusion protein in a cell, using a set of at least a first and a second molecular probe, each probe capable of recognizing a binding site positioned at opposite sides of the fusion region of said fusion protein, comprising the steps of:
- providing a set of probes according to any one of claims 1-7;
- providing a sample comprising a cell
- contacting said sample with said set of probes,
- under conditions that allow juxtaposing said probes on said fusion protein
- contacting said probes with a bridging substance comprising a nucleic acid sequence capable of binding specifically to at least part of the reactive group of said first probe and to at least part of the reactive group of said second probe, and
- detecting juxtaposition of said probes via FRET to determine the presence of said fusion protein.

11. A method according to claim 10, wherein said fusion protein is a tumor-specific fusion protein.

12. A method for detecting at least two interacting molecules in a cell using a set of at least a first and a second molecular probe, each probe comprising a binding domain capable of specifically binding to a different interacting molecule of interest, comprising the steps of:
- providing a set of probes according to any one of claims 1-7,
- providing a sample comprising a cell,
- contacting said sample with said set of probes under conditions that allow juxtaposing said probes on said interacting molecules
- contacting said probes with a bridging substance comprising an oligonucleotide sequence capable of binding specifically to at least part of the reactive group of said first probe and to at least part of the reactive group of said second probe, and
- detecting juxtaposition of said probes via FRET to detect said interacting molecules.

13. A method according to claim 12, wherein at least one of said interacting molecules is a proteinaceous substance, a nucleic acid, a lipid molecule, or a carbohydrate.

14. A method according to any one of claims 10 - 13, wherein said reactive group and bridging substance comprise a DNA, RNA, LNA or PNA sequence.

15. A method according to anyone of claim 10 to 14, including staining said sample for at least one cellular marker to define a target cell population comprising contacting said sample with a compound capable of selectively binding to said cellular marker, wherein said cellular marker is preferably a cluster of differentiation (CD) antigen.

16. A method according to anyone of claims 10-15, comprising FRET detection at the single cell level, preferably using flow cytometry.

17. A diagnostic kit comprising a set of probes according to anyone of claims 1 to 7, preferably in addition comprising a bridging substance comprising an oligonucleotide sequence capable of binding specifically to at least part of the reactive group of said first probe and to at least part of the reactive group of said second probe.

18. Diagnostic kit according to claim 17, wherein said bridging substance is a DNA, RNA, LNA or PNA sequence, preferably wherein both the oligonucleotide reactive groups and the bridging substance are LNA or PNA sequences.

19. Use of a probe set according to anyone of claims 1 to 7, a method according to any one of claims 10 to 16, or a kit according to claim 17, before, during and after treatment of a disease to evaluate the effectiveness of said treatment or to diagnose and / or classify a disease.

## Patentansprüche

1. Set von mindestens einer ersten und einer zweiten molekularen Sonde, wobei jede Sonde in der Lage ist, spezifisch an ein Molekül von Interesse mittels ihrer Bindungsdomäne zu binden, wobei jede Sonde mit einem Farbstoff versehen ist, wobei die Farbstoffe zusammen eine Energieübertragung ermöglichen, wobei jede Sonde zusätzlich mit einer reaktiven Gruppe ausgestattet ist, die das Nebeneinanderlagern der mindestens ersten und zweiten Sonde erlaubt, wobei die reaktive Gruppe für das Modulieren der räumlichen Organisation von nebeneinandergestellten Sonden verfügbar bleibt, nachdem die Sonde an ein Molekül von Interesse gebunden wurde, wobei die reaktive Gruppe ein Oligonukleotid umfasst und wobei die reaktive Gruppe der ersten Sonde nicht direkt mit der reaktiven Gruppe der zweiten Sonde reaktiv ist und eine Überbrückungssubstanz erfordert, die in der Lage ist, mindestens zwei reaktive Gruppen zu Überbrücken, um ein enges Nebeneinanderlagern der Sonden zu vermitteln und/oder zu verbessern.

2. Set gemäß Anspruch 1, wobei die Oligonukleotid-reaktive Gruppe der ersten und zweiten Sonde unabhängig voneinander aus der Gruppe gewählt sind, die aus Desoxyribonukleotid (DNA) - Oligomer, Oxyribonukleotid (RNA) - Oligomer, "Locked Nucleic Acid" (LNA®) und Peptidnukleinsäure (PNA) - Oligomer besteht.

3. Set gemäß Anspruch 1 oder 2, wobei der Farbstoff an die reaktive Gruppe konjugiert ist.

4. Set gemäß einem der Ansprüche 1 bis 3, wobei jede der Sonden mit einer Vielzahl an reaktiven Gruppen ausgestattet ist.

5. Set gemäß einem der Ansprüche 1 bis 4, wobei die Sonde ein Antikörper oder ein funktionell dazu äquivalentes Bindungsfragment ist.

6. Set gemäß einem der Ansprüche 1 bis 5, wobei mindestens einer der Farbstoffe ein Fluorochrom ist.

7. Set gemäß Anspruch 6, wobei das Fluorochrom aus der Gruppe gewählt ist, die aus Fluoresceinisothiocyanat (FITC), Tetramethylrhodaminisothiocyanat (TRITC), Texas-Rot (TR), R-Phycoerythrin (R-PE), Allophycocyanin (APC), Vertretern der Phycobiliproteine, Cyaninfarbstoffen, wie Cy3, Cy5, Cy5.5 oder Cy7, Alexa-Fluor-Farbstoffen, Fluorescein, LightCycler-Farbstoffen, wie etwa LCRed640 oder LCRed705, Tandemkonjugaten dieser Fluorochrome und Quantenpunktfarbstoffen besteht.

8. Verfahren für die Bereitstellung eines Sets von Sonden gemäß einem der Ansprüche 1 - 7, umfassend das Kontaktieren jeder Sonde mit einem geeigneten Farbstoff mit einer reaktiven Gruppe, die ein Oligonukleotid umfasst, zur Bildung eines Konjugats zwischen der Sonde, dem Farbstoff und der reaktiven Gruppe.

9. Verfahren gemäß Anspruch 8, umfassend das Konjugieren des Farbstoffs an die reaktive Gruppe.

10. Verfahren zum Detektieren des Vorhandenseins eines Fusionsproteins in einer Zelle unter Verwendung eines Sets von mindestens einer ersten und einer zweiten molekularen Sonde, wobei jede Sonde in der Lage ist, eine Bindungsstelle zu erkennen, die an gegenüberliegenden Seiten der Fusionsregion des Fusionsproteins positioniert ist, umfassend die folgenden Schritte:
- Bereitstellen eines Sets von Sonden gemäß einem der Ansprüche 1 - 7;
- Bereitstellen einer Probe, die eine Zelle umfasst;
- Kontaktieren der Probe mit dem Set von Sonden;
- unter Bedingungen, die das Nebeneinanderlagern der Sonden auf dem Fusionsprotein erlauben;
- Kontaktieren der Sonden mit einer Überbrückungssubstanz, die eine Nukleinsäuresequenz umfasst, die in der Lage ist, spezifisch an mindestens einen Teil der reaktiven Gruppe der ersten Sonde und an mindestens einen Teil der reaktiven Gruppe der zweiten Sonde zu binden; und
- Detektieren der Nebeneinanderlagerung der Sonden mittels FRET zur Bestimmung des Vorhandenseins des Fusionsproteins.

11. Verfahren gemäß Anspruch 10, wobei das Fusionsprotein ein tumorspezifisches Fusionsprotein ist.

12. Verfahren zum Detektieren von mindestens zwei wechselwirkenden Molekülen in einer Zelle mit Hilfe eines Sets von mindestens einer ersten und einer zweiten molekulare Sonde, wobei jede Sonde eine Bindungsdomäne umfasst, die in der Lage ist, spezifisch an ein unterschiedliches wechselwirkendes Molekül von Interesse zu binden, umfassend die folgenden Schritte:
- Bereitstellen eines Sets von Sonden gemäß einem der Ansprüche 1 - 7;
- Bereitstellen einer Probe, die eine Zelle umfasst;
- Kontaktieren der Probe mit dem Set von Sonden unter Bedingungen, die das Nebeneinanderlagern der Sonden auf den wechselwirkenden Molekülen erlauben;
- Kontaktieren der Sonden mit einer Überbrückungssubstanz, die eine Oligonukleotidsequenz umfasst, die in der Lage ist, spezifisch an mindestens einen Teil der reaktiven Gruppe der ersten Sonde und an mindestens einen Teil der reaktiven Gruppe der zweiten Sonde zu binden, und
- Detektieren der Nebeneinanderlagerung der Sonden mittels FRET zur Detektion der wechselwirkenden Moleküle.

13. Verfahren gemäß Anspruch 12, wobei mindestens eines der wechselwirkenden Moleküle eine proteinartige Substanz, eine Nukleinsäure, ein Lipidmolekül oder ein Kohlenhydrat ist.

14. Verfahren gemäß einem der Ansprüche 10 - 13, wobei die reaktive Gruppe und die Überbrückungssubstanz eine DNA-, RNA-, LNA- oder PNA-Sequenz umfassen.

15. Verfahren gemäß einem der Ansprüche 10 bis 14, welches das Einfärben der Probe für mindestens einen zellulären Marker einschließt, um eine Zielzellenpopulation zu definieren, umfassend das Kontaktieren der Probe mit einer Verbindung, die zum selektiven Binden an den zellulären Marker fähig ist, wobei der zelluläre Marker vorzugsweise ein Cluster vom Differenzierungsantigen (CD) ist.

16. Verfahren gemäß einem der Ansprüche 10 - 15, umfassend eine FRET-Detektion auf Einzelzellenebene, vorzugsweise unter Anwendung von Durchflusszytometrie.

17. Diagnose-Kit, umfassend ein Set von Sonden gemäß einem der Ansprüche 1 bis 7, vorzugsweise außerdem umfassend eine Überbrückungssubstanz, die eine Oligonukleotidsequenz umfasst, die in der Lage ist, spezifisch an zumindest einen Teil der reaktiven Gruppe der ersten Sonde und an zumindest einen Teil der reaktiven Gruppe der zweiten Sonde zu binden.

18. Diagnose-Kit gemäß Anspruch 17, wobei die Überbrückungssubstanz eine DNA-, RNA-, LNA- oder PNA-Sequenz ist, wobei vorzugsweise sowohl die Oligonukleotid-reaktive Gruppe als auch die Überbrückungssubstanz LNA- oder PNA-Sequenzen sind.

19. Verwendung einer Sonde gemäß einem der Ansprüche 1 bis 7, eines Verfahrens gemäß einem der Ansprüche 10 bis 16 oder eines Kits gemäß Anspruch 17 vor, während und nach der Behandlung einer Erkrankung, um die Wirksamkeit der Behandlung zu evaluieren oder um eine Erkrankung zu diagnostizieren und/oder zu klassifizieren.

## Revendications

1. Ensemble d'au moins une première et une deuxième sondes moléculaires, chaque sonde étant capable de se lier d'une manière spécifique à une molécule d'intérêt par l'intermédiaire de son domaine de liaison, chaque sonde étant pourvue d'un colorant, lesdits colorants permettant ensemble un transfert d'énergie, chaque sonde étant en outre pourvue d'un groupe réactif permettant la juxtaposition desdites au moins première et deuxième sondes, ledit groupe réactif restant disponible pour la modulation de l'organisation spatiale de sondes juxtaposées après que la sonde s'est liée à une molécule d'intérêt, ledit groupe réactif comprenant un oligonucléotide, et le groupe réactif de ladite première sonde n'étant pas directement réactif vis-à-vis du groupe réactif de ladite deuxième sonde et exigeant une substance pontante capable de ponter au moins deux groupes réactifs dans le but de médier et/ou de renforcer une juxtaposition étroite desdites sondes.

2. Ensemble selon la revendication 1, dans lequel le groupe réactif oligonucléotide de ladite première et de ladite deuxième sondes est indépendamment choisi dans le groupe consistant en un désoxyribonucléotide (ADN) oligomère, un oxyribonucléotide (ARN) oligomère, un acide nucléique verrouillé (LNA®) et un acide nucléique peptidique (PNA) oligomère.

3. Ensemble selon la revendication 1 ou 2, dans lequel le colorant est conjugué au groupe réactif.

4. Ensemble selon l'une quelconque des revendications 1 à 3, dans lequel chacune desdites sondes est pourvue d'une multiplicité de groupes réactifs.

5. Ensemble selon l'une quelconque des revendications 1 à 4, dans lequel ladite sonde est un anticorps ou un fragment de liaison qui lui est fonctionnellement équivalent.

6. Ensemble selon l'une quelconque des revendications 1 à 5, dans lequel ledit au moins un desdits colorants est un fluorochrome.

7. Ensemble selon la revendication 6, dans lequel ledit fluorochrome est choisi dans le groupe consistant en l'isothiocyanate de fluorescéine (FITC), l'isothiocyanate de tétraméthylrhodamine (TRITC), le rouge Texas (TR), la R-phycoérythrine (R-PE), l'allophycocyanine (APC), les membres des phycobiliprotéines, les colorants de cyanine tels que le Cy3, le Cy5, le Cy5.5 ou le Cy7, les colorants Alexa Fluor, la fluorescéine, les colorants LightCycler, tels que le LCRed640 ou le LCRed705, les conjugués en tandem de ces fluorochromes, et les colorants à point quantique.

8. Procédé pour mettre à disposition un ensemble de sondes selon l'une quelconque des revendications 1 à 7, comprenant la mise en contact de chaque sonde avec un colorant approprié et avec un groupe réactif comprenant un oligonucléotide pour former un conjugué entre ladite sonde, ledit colorant et ledit groupe réactif.

9. Procédé selon la revendication 8, comprenant la conjugaison du colorant au groupe réactif.

10. Procédé pour détecter la présence d'une protéine de fusion dans une cellule, par utilisation d'un ensemble d'au moins une première et une deuxième sondes moléculaires, chaque sonde étant capable de reconnaître un site de liaison positionné sur les côtés opposés de la région de fusion de ladite protéine de fusion, comprenant les étapes de
- mise à disposition d'un ensemble de sondes selon l'une quelconque des revendications 1 à 7 ;
- mise à disposition d'un échantillon comprenant une cellule,
- mise en contact dudit échantillon avec ledit ensemble de sondes,
- dans des conditions qui permettent la juxtaposition desdites sondes sur ladite protéine de fusion,
- mise en contact desdites sondes avec une substance pontante comprenant une séquence d'acide nucléique capable de se lier d'une manière spécifique à au moins une partie du groupe réactif de ladite première sonde et à au moins une partie du groupe réactif de ladite deuxième sonde, et
- détection de la juxtaposition desdites sondes par FRET pour déterminer la présence de ladite protéine de fusion.

11. Procédé selon la revendication 10, dans lequel ladite protéine de fusion est une protéine de fusion spécifique de tumeur.

12. Procédé pour détecter la présence d'au moins deux molécules interagissantes dans une cellule par utilisation d'un ensemble d'au moins une première et une deuxième sondes moléculaires, chaque sonde comprenant un domaine de liaison capable de se lier d'une manière spécifique à une molécule interagissante d'intérêt différente, comprenant les étapes de :
- mise à disposition d'un ensemble de sondes selon l'une quelconque des revendications 1 à 7,
- mise à disposition d'un échantillon comprenant une cellule,
- mise en contact dudit échantillon avec ledit ensemble de sondes dans des conditions qui permettent la juxtaposition desdites sondes sur lesdites molécules interagissantes,
- mise en contact desdites sondes avec une substance pontante comprenant une séquence oligonucléotidique capable de se lier d'une manière spécifique à au moins une partie du groupe réactif de ladite première sonde et à au moins une partie du groupe réactif de ladite deuxième sonde, et
- détection de la juxtaposition desdites sondes par FRET pour détecter lesdites molécules interagissantes.

13. Procédé selon la revendication 12, dans lequel au moins l'une desdites molécules interagissantes est une substance protéinée, un acide nucléique, une molécule lipidique ou un hydrate de carbone.

14. Procédé selon l'une quelconque des revendications 10 à 13, dans lequel ledit groupe réactif et ladite substance pontante comprennent une séquence d'ADN, d'ARN, de LNA ou de PNA.

15. Procédé selon l'une quelconque des revendications 10 à 14, comprenant la coloration dudit échantillon pour au moins un marqueur cellulaire pour définir une population de cellules cibles, comprenant la mise en contact dudit échantillon avec un composé capable de se lier d'une manière sélective audit marqueur cellulaire, ledit marqueur cellulaire étant de préférence un antigène cluster de différenciation (CD).

16. Procédé selon l'une quelconque des revendications 10 à 15, comprenant la détection par FRET au niveau d'une cellule individuelle, de préférence par utilisation d'une cytométrie en flux.

17. Trousse de diagnostic comprenant un ensemble de sondes selon l'une quelconque des revendications 1 à 7, comprenant de préférence, en outre, une substance pontante comprenant une séquence oligonucléotidique capable de se lier d'une manière spécifique à au moins une partie du groupe réactif de ladite première sonde et à au moins une partie du groupe réactif de ladite deuxième sonde.

18. Trousse de diagnostic selon la revendication 17, dans laquelle ladite substance pontante est une séquence d'ADN, d'ARN, de LNA ou de PNA, de préférence dans laquelle les groupes réactifs oligonucléotidiques et la substance pontante sont des séquences de LNA ou de PNA.

19. Utilisation d'un ensemble de sondes selon l'une quelconque des revendications 1 à 7, d'un procédé selon l'une quelconque des revendications 10 à 16 ou d'une trousse selon la revendication 17, avant, pendant et après traitement d'une maladie, pour évaluer l'efficacité dudit traitement ou pour diagnostiquer et/ou classifier une maladie.
